# EUROPEAN PATENT SPECIFICATION

(11) **EP 2 405 829 B1**
(45) Date of publication and mention of the grant of the patent: **17.04.2013**
(21) Application number: 10710640.3
(22) Date of filing: 04.03.2010
(51) Int. Cl.: A61B 17/12, A61B 17/00

(54) **A DEVICE FOR STRETCHING A PLASTIC RING**
VORRICHTUNG ZUM DEHNEN EINES ELASTISCHEN RINGS
DISPOSITIF PERMETTANT D'ÉTIRER UN ANNEAU ÉLASTIQUE

(30) Priority: 12.03.2009 IT RE20090023
(43) Date of publication of application: 18.01.2012
(73) Proprietor: THD S.p.A., 42015 Correggio, Reggio Emilia (IT)
(72) Inventor: BASTIA, Filippo, I-41012 Carpi (modena) (IT)
(74) Representative: Colò, Chiara
(86) International application number: PCT/IB2010/050932
(87) International publication number: WO 2010/103434

(56) References cited:
- WO-A-00/03642
- DE-U1- 9 205 453
- US-B1- 6 482 213

## Description

### Technical Field

The invention relates to a device for stretching an elastic ring, and is particularly applicable for treatment of haemorrhoidal complaints.

### Background Art

The prior art describes pistol-type guns for releasing elastic rings (rubber bands) comprising a command tube connected to a gripping handle for manoeuvring, supporting one or more rubber rings to be released.

The command tube comprises an internal tube on a free end of which the rubber ring is predisposed, and an external tube moved in advancement with respect to the internal tube in order to determine release of the rubber ring. The handle is fixed to the internal tube and exhibits a trigger which can be manually activated by an operator in order to command the advancement of the external tube.

For loading the rubber rings, substantially conical tips are known, which are designed to be coupled on the tip of the releasing piston of the rubber ring.

An example of these devices is described in patent DE9205453, which illustrates a device for stretching a rubber ring.

The device comprises a tip, exhibiting at least a conical tract and a cylindrical tract, provided with a plurality of sliding guides.

The device is completed by pushing means slidable along the tip by means of a plurality of ribs that are translatable internally of the corresponding plurality of guides.

Devices of the above-described type exhibit some drawbacks.

Firstly the geometric conformation of the tip requires special attention by the operator during the loading stage.

The application of the ring requires complete sliding of the pushing means from the start to the end of the tip in order to force the ring to stretch.

As it has to cross progressively-increasing transversal sections of the tip, the rubber ring requires a prolonged application of the pushing means to reach the housing seating.

If there should be a distancing of the pushing means from the rubber ring, due for example to a moment of distraction on the part of the user, the ring slides back from the previously-assumed position.

In the same way, even the initial portion of the tip exhibits drawbacks as far as the ring loading operations are concerned.

The operator performing the pre-loading of the ring on the first cylindrical tract of the tip might find that the ring frees itself of its positioning and falls to earth, which means the operator will have to take a new ring and perform the loading operation once more.

Also, the fact that the terminal portion of the tip is provided with a first and a second cylindrical tracts, the second having a smaller diameter than the first which is destined to insert into the release pistol, constitutes a drawback for the operations of locating the ring in the housing seating afforded on the release device.

The operator who is abuttingly locating the end of the releasing device on the broadening of section of the two cylindrical tracts might, due to a distancing of the tip, cause the rubber ring not to abut correctly on the release pistol, but rather have it fall onto the second cylindrical tract.

In the above-described situation, the operation of recuperating the rubber ring is disadvantageously difficult for the operator to perform, as the ring is solidly anchored to the cylindrical tract of the tip.

WO 00/03642 discloses a mounting apparatus for mounting an endless cord which is expandable from a contracted condition to an expanded condition onto an end of a structure having a transverse dimension greater than that of the cord when in the contracted condition. The mounting apparatus comprises a tapered adaptor for the cord to be propelled over onto the end of the structure, having a forward smaller end for location in the cord in its contracted condition and a rear larger end for juxtaposing with the end of the structure. The mounting apparatus further comprises an expander device movable relative to the adapter to propel the cord over the adaptor onto the rear larger end thereof.

In this context, the technical objective underlying the present invention is to provide a device for stretching an elastic-material (rubber) ring which obviates the drawbacks in the above-cited prior art.

In particular, an aim of the present invention is to provide a device for stretching an elastic ring which facilitates loading operations of the rubber ring.

A further aim of the present invention is to provide a device for stretching a rubber ring which facilitates the operator's work during the pre-loading stage.

A further aim of the present invention is to provide a device for stretching a rubber ring which provides the operator with a facilitated loading of the ring on the release pistol.

A further aim of the present invention is to provide a device for stretching a rubber ring which, in a case of wrong loading of the ring, enables the operator to recuperate the ring simply and immediately.

The set technical objective and the specified aims are substantially attained by a device for stretching a rubber ring comprising the technical characteristics set out in one or more of the appended claims.

### Disclosure of Invention

The present invention is defined in claim 1.

Further characteristics and advantages of the present invention will better emerge from the non-limiting description that follows of a preferred but not exclusive embodiment of a device for stretching a rubber ring.

A device for stretching a rubber ring of the present invention will be disclosed below.

The device is applicable in loading rubber rings on a release piston for legating tissues such as, for example, the anal mucosa of a patient.

The invention is advantageously applied in treatment of haemorrhoidal complaints related to the mucosa.

The device comprises a loading body and pushing means, the pushing means being slidable on the body in order to load at least a rubber ring thereon.

The pushing means comprise a hollow body internally of which a plurality of ribs develop.

The hollow body preferably exhibits a tubular shape.

The hollow body exhibits an internal diameter which is greater than the external diameter of the loading body in order to facilitate sliding of the loading body internally of the pushing means.

The plurality of ribs exhibits, starting from the inside of the hollow body, a radial development towards the centre of the hollow body.

The plurality of ribs is uniformly spaced over the circumference of the hollow body, preferably resulting in at least four ribs.

The four ribs are staggered by 90 degrees to one another over the circumference of the hollow body.

Each rib has a substantially rectangular edge terminating on a portion which faces the loading body with a respective peak.

Each peak enters into contact with the elastic ring and, during the rising thereof along the loading body, collaborates with respective grooves afforded on the loading body and more fully described herein below, to push the ring.

The loading body exhibits a development direction along a prevalent direction X-X.

The loading body exhibits a plurality of grooves, each having an orientation which is parallel to the prevalent direction X-X in order to facilitate sliding, along direction X-X, of the plurality of ribs of the pushing means.

Each groove of the plurality of grooves exhibits a radial progression.

Each groove of the plurality of grooves extends uniformly on the loading body and is preferably spaced from a preceding and a following groove by 90 degrees.

The loading body comprises a substantially conical first portion, a second portion, located downstream of the first portion, and a cylindrical portion, located downstream of the second portion.

The substantially conical first portion exhibits a base having an external diameter D and an apex.

The proximal end exhibits an external diameter D' and the distal end exhibits an external diameter d.

The external diameter D' of the proximal end is greater than the external diameter d of the distal end.

The second portion exhibits a frustoconical shape.

However, without forsaking the ambit of protection of the following invention, the second portion exhibits, starting from the external diameter D of the base of the first substantially conical zone, a section having a decreasing progression.

In other words the second portion exhibits, from the proximal end to the distal end, a tapering transversal section.

The proximal end of the second conical portion faces the first substantially conical portion.

The distal end of the first portion is couplable to a tube of a releasing pistol for legating tissues.

The coupling mode will be more fully explained herein below.

The proximal end is contiguous to the base and the external diameter D of the base exhibits a same dimension as the external diameter D' of the proximal end.

More precisely, the external diameter D of the base coincides with the external diameter D' of the proximal end.

In this way the loading of the rubber ring on the body is much easier.

In fact, the rubber ring, having crossed the substantially conical first portion, by means of the pushing of the pushing means and by effect of its own elastic force, autonomously translates along the second portion up to reaching the housing seating of the tubular body.

Consequently the pushing means are only operative for a tract of the run thereof along the loading body, i.e. they are operative up until the rubber ring has crossed the external diameter D' of the second tract.

The conformation of the plurality of grooves enables however the sliding of the pushing means along the whole loading body.

This is in order to enable the operator to perform the loading, in a traditional way, without seeing the device and thus enabling the operator to focus her or his attention on the patient.

With reference to the plurality of grooves, each groove exhibits an extension over the whole loading body.

More precisely, each groove belonging to the plurality of grooves exhibits a first end located in proximity of the apex of the substantially conical first portion, and a second end terminating in proximity of the cylindrical portion.

At least one of the grooves exhibits an abutting element terminating before the second end of the remaining grooves.

In this way it is possible to block the course of the pushing means with respect to the loading body by providing an endrun stop for the pushing means.

Similarly the two components can be pre-assembled with a constraint between the two components such that when the device is received the loading body or the pushing means is constrained to the relative component.

The second portion exhibits a seating at the distal end.

The seating, which is substantially annular, functions as a housing seating of the tube of a pistol for elastic ligature of tissues.

The second distal end of the second portion is co-penetrable by the tube.

In this way the distal end and the tube of the pistol do not generate any intermediate space, such that the rubber ring is facilitated to position on periphery of the tube.

If there is an accidental detachment of the loading body from the tube, the rubber ring, by action of the pushing means, tends to position on the cylindrical portion.

Thanks to the prolonged extension of the plurality of guides, the operator, following the run of the pushing means, pushes the rubber ring along the cylindrical body and thus obtains an easy recuperation of the rubber ring. The substantially conical first portion thus exhibits the pre-loading means. The pre-loading means, located between the base and the apex, are defined by a broadening of section of the substantially conical first portion.

The pre-loading means are more preferably located in proximity of the apex.

The pre-loading means are preferably defined by an ogive body.

In this way the operator can insert the rubber ring onto the loading body, locating it downstream of the pre-loading means, and thereafter apply thereto the pushing means in order to complete the loading of the rubber ring onto the pistol.

In the same way, the pre-loading means enable the device to be made for a single-use application.

The device could be distributed directly by the producer with the rubber ring already installed on the loading body and located downstream of the pre-loading means.

In this way the operator is able to load the rubber ring by sliding the pushing means along the loading body and thus without touching the rubber ring.

The device for stretching a rubber ring exhibits a further embodiment. The substantially conical first portion is defined by a first plurality of risers. Each riser is uniformly subdivided and surrounds a corresponding groove. Seen from the rear, i.e. with the apex, as it were, entering the sheet, the plurality of risers surrounds a cross, the cross being defined in negative by the plurality of grooves.

In other words the plurality of risers defines pairs of L shapes, each arranged specularly to a contiguous pair with respect to an imaginary horizontal or vertical plane.

The first plurality of risers defines first generatrices of the conical portion.

In the same way, the second portion too is defined by a second plurality of risers.

The second plurality of risers preferably follows the first plurality and exhibits a same arrangement thereas.

The second plurality of risers also defines second generatrices of the truncoconical second portion.

The first plurality of risers and the second plurality of risers internally define recesses for lightening the loading body.

The recesses not only lighten the loading body but also reduce the contact surface of the rubber ring, during the sliding thereof, along the loading body.

The functioning of the device is the following.

The operator collects a rubber ring by inserting the apex of the first conical portion internally of the rubber ring.

The operator then pushes the rubber ring along the first conical portion until the ring moves past the pre-loading means.

In a further embodiment, the loading body can already exhibit a rubber ring located downstream of the pre-loading means.

Thereafter the operator proceeds to couple the loading body on the legating pistol.

During this stage it is sufficient to proceed to coupling the cylindrical portion internally of the tube of the pistol.

When the tube strikes against the seating, present on the second portion, the pistol is ready to be loaded with the rubber ring.

During the subsequent stage the operator takes the pushing means and slides them along the loading body, and pushes the ring up to when it has crossed the substantially conical first portion.

As soon as the ring passes the external diameter D' of the second portion, thanks to the elastic properties thereof it will autonomously slide on the whole second portion, stopping on the tube without any further manoeuvring on the part of the operator.

The invention attains the set aims, and obviates the drawbacks noted in the prior art.

Primarily, the conformation of the loading body enables an easy loading of the rubber ring on the tube of the legating pistol.

The arrangement of a section having a decreasing section downstream of the substantially conical first portion enables the ring, after having gone past the conical portion, to translate autonomously up to being positioned on the tube.

The seating located on the distal end drastically reduces the occurrence of loading errors of the rubber ring on the tube.

The portion of the tube, couplable internally of the seating, enables a continuity of surface to be obtained between the loading body and the tube, preventing the rubber ring from becoming positioned on the cylindrical terminal portion.

Even should the rubber ring erroneously become located on the cylindrical portion, the elongate conformation of the grooves, i.e. crossing the whole loading body, enables easy recuperation of the rubber ring by simply prolonging the action of the pushing means on the body.

The presence of the pre-loading means facilitates the loading operations of the rubber ring on the loading body.

Once the ring has been manually inserted and pushed downstream of the pre-loading means, the loading body can be manoeuvred without the ring being newly disconnected therefrom.

In the same way the presence of the pre-loading means enables a single-use device for stretching a rubber ring to be realised.

Finally, the plurality of risers of the second embodiment of the loading body drastically reduces the external surface thereof and consequently the contact surface with the rubber ring.

This facilitates a reduction in friction during the loading operation and consequently a reduction in the tensional stresses to which the rubber ring is subjected during the loading operation.

## Claims

1. A device for stretching an elastic ring, comprising:
pushing means defined by a hollow body internally of which a plurality of ribs develop;
a loading body for at least a rubber band, the loading body having a prevalent development along a direction and exhibiting a plurality of grooves for facilitating sliding along the direction of the plurality of ribs on the corresponding plurality of grooves; the loading body comprising:
a first substantially conical portion exhibiting a base, having an external diameter and an apex;
a second portion exhibiting a proximal end, having an external diameter and a distal end, having an external diameter; the external diameter of the proximal end being greater than the external diameter of the distal end;
the proximal end being contiguous to the base and the external diameter of the base exhibiting a same length as the external diameter of the proximal end;
**characterized in that** the second portion is frustoconical, such that
during the loading of the rubber band by the pushing means, first a stretching of the rubber band along the first substantially-conical portion followed by an elastic returning of the rubber band along the second portion is facilitated.

2. The device of claim 1, wherein it further comprises a cylindrical portion located distal of the second portion.

3. The device of claim 2, wherein the plurality of grooves extends along all of the loading body.

4. The device of claim 2, wherein each groove belonging to the plurality of grooves exhibits a first end which is located in proximity of the apex and a second end which terminates in proximity of the cylindrical portion, for facilitating sliding of the pushing means throughout the whole extension of the first conical portion and the second portion.

5. The device of claim 1, wherein the distal end exhibits a seating for facilitating housing internally thereof of a tube of a pistol for elastic ligature of tissues.

6. The device of claim 1, wherein it further comprises means for pre-loading, defined by a broadening of section of the first substantially conical portion located between the apex and the base preferably in proximity of the apex.

7. The device of claim 6, wherein the means for pre-loading are defined by an ogive cone body.

8. The device of claim 1, wherein the substantially-conical first portion is defined by a first plurality of risers defining first generatrices of the conical portion.

9. The device of claim 8, wherein the second portion is defined by a second plurality of risers defining second generatrices of the frustoconical second portion.

10. The device of claims 8 and 9, wherein the first plurality of risers and the second plurality of risers internally define respective recesses for lightening the loading body.

## Patentansprüche

1. Vorrichtung zum Dehnen eines elastischen Rings, umfassend:
Schiebemittel, definiert durch einen Hohlkörper, in dessen Innenraum sich eine Vielzahl von Rippen entwickeln;
einen Ladekörper für mindestens ein Gummiband, wobei der Ladekörper eine vorwiegende Entwicklung entlang einer Richtung besitzt und eine Vielzahl von Rillen aufweist, um das Gleiten entlang der Richtung der Vielzahl von Rippen auf der entsprechenden Vielzahl von Rillen zu erleichtern, wobei der Ladekörper umfasst:
einen ersten im Wesentlichen kegelförmigen Abschnitt, aufweisend eine Basis, besitzend einen Außendurchmesser und eine Kegelspitze;
einen zweiten Abschnitt, aufweisend ein proximales Ende, besitzend einen Außendurchmesser und ein distales Ende, besitzend einen Außendurchmesser, wobei der Außendurchmesser des proximalen Endes größer ist als der Außendurchmesser des distalen Endes;
wobei das proximale Ende angrenzend an die Basis angeordnet ist und der Außendurchmesser der Basis eine selbe Länge wie der Außendurchmesser des proximalen Endes aufweist;
**dadurch gekennzeichnet, dass** der zweite Abschnitt kegelstumpfförmig ausgebildet ist, sodass während des Ladens des Gummibands durch die Schiebemittel zuerst ein Dehnen des Gummibands entlang des ersten im Wesentlichen kegelförmigen Abschnitts, gefolgt von einer elastischen Rückfederung des Gummibands entlang des zweiten Abschnitts, erleichtert wird.

2. Vorrichtung nach Anspruch 1, wobei sie zudem einen zylindrischen Abschnitt, angeordnet distal zum zweiten Abschnitt, umfasst.

3. Vorrichtung nach Anspruch 2, wobei sich die Vielzahl von Rillen entlang des gesamten Ladekörpers erstreckt.

4. Vorrichtung nach Anspruch 2, wobei jede zur Vielzahl der Rillen gehörende Rille ein erstes Ende aufweist, das sich in der Nähe der Kegelspitze befindet, und ein zweites Ende, das in der Nähe des zylindrischen Abschnitts endet, um das Gleiten der Schiebemittel durch die gesamte Ausdehnung des ersten kegelförmigen Abschnitts und des zweiten Abschnitts zu erleichtern.

5. Vorrichtung nach Anspruch 1, wobei das distale Ende einen Sitz aufweist, um die Aufnahme eines Rohrs einer Pistole für die elastische Ligatur von Gewebe im Innenraum zu erleichtern.

6. Vorrichtung nach Anspruch 1, wobei sie zudem Mittel zum Vorladen umfasst, definiert durch eine Querschnittsverbreiterung des ersten im Wesentlichen kegelförmigen Abschnitts, angeordnet zwischen der Kegelspitze und der Basis, vorzugsweise in der Nähe der Kegelspitze.

7. Vorrichtung nach Anspruch 6, wobei die Mittel zum Vorladen durch einen spitzbogigen Konuskörper definiert sind.

8. Vorrichtung nach Anspruch 1, wobei der im Wesentlichen kegelförmige erste Abschnitt durch eine erste Vielzahl von Steigungen definiert ist, definierend erste Erzeugende des kegelförmigen Abschnitts.

9. Vorrichtung nach Anspruch 8, wobei der zweite Abschnitt durch eine zweite Vielzahl von Steigungen definiert ist, definierend zweite Erzeugende des kegelstumpfförmigen zweiten Abschnitts.

10. Vorrichtung nach Anspruch 8 und 9, wobei die erste Vielzahl von Steigungen und die zweite Vielzahl von Steigungen intern jeweilige Vertiefungen für das Leichtermachen des Ladekörpers definieren.

## Revendications

1. Dispositif permettant d'étirer un anneau élastique, comprenant :
des moyens de poussée définis par un corps creux à l'intérieur duquel une pluralité de nervures se développent ;
un corps de chargement pour au moins un élastique, le corps de chargement ayant un développement prévalant le long d'une direction et présentant une pluralité de rainures pour faciliter le coulissement le long de la direction de la pluralité de rainures sur la pluralité de rainures correspondantes ; le corps de chargement comprenant :
une première partie substantiellement conique présentant une base, ayant un diamètre externe et un sommet ;
une seconde partie présentant une extrémité proximale, ayant un diamètre extérieur et une extrémité distale ayant un diamètre extérieur ; le diamètre extérieur de l'extrémité proximale étant supérieur au diamètre extérieur de l'extrémité distale ;
l'extrémité proximale étant contiguë à la base et le diamètre extérieur de la base présentant une même longueur que le diamètre extérieur de l'extrémité proximale ;
**caractérisé en ce que** la seconde partie possède une forme troncoconique, de sorte que lors du chargement de l'élastique par les moyens de poussée, un premier étirement de l'élastique, le long de la première partie substantiellement conique suivi par un retour élastique de l'élastique le long de la seconde partie, est facilité.

2. Dispositif selon la revendication 1, **caractérisé en ce qu'**il comprend de plus une partie cylindrique située de façon distale par rapport à la seconde partie.

3. Dispositif selon la revendication 2, dans lequel la pluralité de rainures se développent tout le long du corps de chargement.

4. Dispositif selon la revendication 2, dans lequel chaque rainure appartenant à la pluralité de rainures présente une première extrémité étant située à proximité du sommet et une seconde extrémité se terminant à proximité de la partie cylindrique, pour faciliter le coulissement des moyens de poussée dans toute la totalité du développement de la première partie conique et de la seconde partie.

5. Dispositif selon la revendication 1, dans lequel l'extrémité distale présente un logement pour faciliter l'installation à l'intérieur dudit logement d'un tube d'un pistolet destiné à la ligature élastique de tissus.

6. Dispositif selon la revendication 1, **caractérisé en ce qu'**il comprend de plus des moyens de pré-chargement, définis par un élargissement de la section de la première partie substantiellement conique située entre le sommet et la base, de préférence à proximité du sommet.

7. Dispositif selon la revendication 6, dans lequel les moyens de pré-chargement sont définis par un corps en ogive.

8. Dispositif selon la revendication 1, dans lequel la première partie substantiellement conique est définie par une première pluralité d'élévations définissant les premières génératrices de la partie conique.

9. Dispositif selon la revendication 8, dans lequel la seconde partie est définie par une seconde pluralité d'élévations définissant les secondes génératrices de la seconde partie troncoconique.

10. Dispositif selon les revendications 8 et 9, dans lequel la première et la seconde pluralité d'élévations définissent à l'intérieur des renfoncements respectifs pour alléger le corps de chargement.
